# EUROPEAN PATENT APPLICATION

(11) **EP 4 353 225 A1**
(43) Date of publication of application: **17.04.2024**
(21) Application number: 23202880.3
(22) Date of filing: 11.10.2023
(51) Int. Cl.: A61K 31/164, A61K 36/15, A61K 36/185, A61K 31/353, A61K 31/201, A61K 31/202, A61P 29/00, A61P 13/00, A61P 13/02, A61P 13/08, A61P 13/10, A61P 13/12, A61P 15/02, A23L 33/105

(54) **NUTRACEUTICAL OR PHARMACEUTICAL COMPOSITION FOR THE TREATMENT OF PELVIC PAIN**

(30) Priority: 12.10.2022 IT 202200021057
(71) Applicant: Erbozeta S.p.A., 47894 Chiesanuova (SM)
(72) Inventor: PASTORELLI, Chiara, 47100 FORLI' (IT); ZAVAGLIA, Roberto, 47891 FALCIANO (SM)
(74) Representative: Rambelli, Paolo

(57) **Abstract**

Pharmaceutical or nutraceutical composition comprising palmitoylethanolamide (PEA), dry extract of maritime pine and hemp seed oil substantially free from phytocannabinoids, for use in the treatment of pelvic pain, preferably the composition comprising from 100 to 1200 mg of PEA, from 2.5 to 30 mg of dry extract of maritime pine in quantities of 1.6 to 4.2 mg, and from 12.5 to 150 mg of hemp seed oil substantially free from phytocannabinoids.

## Description

The present invention relates to a pharmaceutical or nutraceutical composition containing as active agents palmitoylethanolamide (PEA), extract of maritime pine or proanthocyanidins, particularly procyanidins from extract of maritime pine, and hemp seed oil or omega 3 and omega 6 fatty acids from hemp seed oil, for use in the treatment of acute, cyclical and chronic pelvic pain.

Pelvic pain is a discomfort in the lower abdomen that is difficult to define as it represents a symptom attributable to numerous disorders and diseases of different origins: gynecological, reproductive, gastrointestinal, urinary, musculoskeletal.

Disorders that may cause pelvic pain may originate from any structure located in the pelvic cavity:
- Urinary tract
- Female and male reproductive organs
- Gastrointestinal system
- Musculoskeletal system
- Vascular system

### Most frequent causes of uro-andrological pelvic pain

- Cystitis (acute, recurrent, chronic; bacterial or abacterial)
- Interstitial cystitis
- Calculosis
- Ureteritis
- Urethritis
- Urethral syndrome
- Nephritis (pyelonephritis, glomerulonephritis, tubulointerstitial nephritis)
- Bladder pain syndrome (BPS)
- Chronic Pelvic Pain Syndrome (CPPS)
- Neurological bladder
- Bladder neck sclerosis
- Neoplasms
- Prostatitis (acute, recurrent, chronic; bacterial or abacterial)
- Prostatodymia

### Most frequent causes of gynecological pelvic pain

- Primary dysmenorrhea
- Endometriosis
- Adenomyosis
- Pelvic inflammatory disease
- Uterine fibromatosis
- Functional somatic syndromes (FSS's): vulvar vestibulitis and vulvodynia
- Dyspareunia
- Cervicitis
- Endometritis
- Neoplasms

Pelvic pain may be:
- acute, when it appears suddenly, it worsens rapidly and then disappears.
- cyclic, often associated with the menstrual cycle.
- chronic, when it persists for more than 3 months.

Chronic pelvic pain is defined by the European Association of Urology (EAU) as chronic or persistent pain felt in pelvic-related structures in men or women. It is often associated with negative cognitive, behavioral, sexual and emotional consequences as well as symptoms indicating urinary, sexual, intestinal, pelvic floor or gynecological dysfunction.

The most frequent cause of chronic pelvic pain is inflammation of the internal organ, with significant activation and degranulation of mast cells (MC). Local inflammation, stimulated by mediators released by mast cells, determines a peripheral sensitization process, with a consequent increase in pain sensitization. The peripheral sensitization is then followed by a process of central sensitization, with an increase in the activity and excitability of the sensory neurons in the spinal cord and in the higher centers, which amplifies the symptoms and tends to persist even when the primitive peripheral stimulus that triggered it disappears, becoming an independent phenomenon, responsible for the chronicity of the symptoms.

According to the most recent data available in the literature, chronic pelvic pain affects, depending on the age group considered, a percentage of women ranging from 5.6% to 30.9% and from 2.0 to 17% in men. Consequently, pelvic pain affects women more than men and represents, regardless of gender, a pathology that severely limits the quality of life and significantly impacts the national healthcare system.

Chronic pain is therefore quite frequent, especially in women, and it is not always possible to identify a well-defined cause; consequently treatment is often aimed at improving the symptoms.

The treatment of chronic pelvic pain involves the use of analgesic, anti-inflammatory and neuromodulatory drugs which may lead to unwanted side effects.

Taking into account the large incidence of pelvic pain on the population and its influence on the quality of life of those affected, it is desirable to provide a composition useful for alleviating both chronic and acute pelvic pain which makes use of natural extracts and raw materials and which may therefore be used both as a pharmaceutical formulation and as a nutraceutical formulation, i.e. which meets the legal requirements to be classified as such.

The maritime pine bark extract (Pinus pinaster Aiton) is rich in oligomeric flavonoids belonging to the procyanidin group which exert important properties: scavenging of oxygen- and nitrogen-free radicals (antioxidant), strong anti-inflammatory activity, ability to stimulate the production of collagen and hyaluronic acid and promote the production of endothelial nitric oxide, which helps improve microcirculation.

Palmitoylethanolamide (PEA) is an endogenous lipid compound (palmitic acid amide) that has analgesic and anti-inflammatory properties and is involved in the release of pro-inflammatory mediators by mast cells, immune cells believed to be involved in the onset and maintenance of neuropathic pain. Scientific studies have demonstrated its effectiveness in the treatment of chronic painful syndromes such as sciatica, peripheral neuropathies, diabetic neuropathies, postherpetic neuralgia but also pelvic pain, osteoarthritis, headaches and postoperative pain.

Hempseed oil, obtained from Cannabis sativa L. seeds, is known for its nutritional and health properties and its bioactivity. Compared to other vegetable oils, it is a particularly rich source of essential fatty acids. Hemp oil is one of the few food sources of linolenic acid. It is well known for the excellent balance of omega 3 and omega 6 essential fatty acids - present in relation to the 3:1 ratio - and for the important antioxidant, immunomodulatory and anti-inflammatory properties with which it is provided. Hemp seed oil is not to be confused with the oil that is extracted from some parts of the plant and in particular the stems, leaves and flowers, which contain cannabinoids. Hemp seed oil, on the other hand, comes exclusively from the pressing of the seeds of the plant. The seeds do not contain cannaboinoids, but have a rich profile of nutrients, fatty acids and bioactive compounds useful for human health.

WO2020/214569 describes an allegedly synergistic composition containing PEA and hemp oil in weight ratios of 1.2:1 to 4.5:1 (PEA/hemp oil) for the treatment of inflammatory and neuropathic pain. The composition uses a hemp oil derived from the aerial parts of the plant which contains significant quantities of phytocannabinoids such as cannabidiol (CBD) and cannabigerol (CBG) as well as terpenes such as beta-caryophyllene (BCP); in particular, compositions are described wherein the weight ratio between PEA and the phytocannabinoid component in the hemp oil is in the range from 50:1 to 30:1.

The presence of phytocannabinoids precludes the use of the composition as a nutraceutical product in accordance with European legislation.

In Italy, the cultivation of Cannabis sativa L. of the varieties permitted for the production of seeds and seed derivatives is conducted pursuant to the law of 2 December 2016, n. 242, containing provisions for the promotion of the cultivation and agro-industrial supply chain of hemp. The cultivation of cannabis plants for the production of leaves and inflorescences or active substances for medicinal use is regulated by the decree of the President of the Republic of 9 October 1990, no. 309, which prohibits the cultivation thereof without the required authorization from the Ministry of Health.

The aim of the present invention is therefore to provide a synergistic composition, specific for the treatment of pelvic pain, both acute and chronic, having a broad spectrum of action on inflammatory cytokines and which may also be used as a nutraceutical composition.

### Summary of the invention

In view of the above-mentioned aims, the object of the invention is a composition as defined in the following claims.

Dosage forms for the oral administration of the composition, particularly solid and liquid pharmaceutical forms for oral use, are also an object of the invention. Further features of the composition according to the invention and its activity are illustrated in the detailed description that follows, with reference to the accompanying drawings.

### Summary of drawings

In the accompanying drawings, referring to the following experimental tests:
- Figure 1 shows the cell viability diagrams of THP-1 differentiated following treatment with micronized PEA (A), maritime pine extract (B), hemp oil (C) and MIX (exemplary composition according to the invention) (D1) *lower cell viability <70%);
- Figure 2 is a histogram showing the nuclear levels of the transcription factor Nf-kB expressed as fold change compared to the control (cells treated with medium conditioned only with the inflammatory stimulus, *E.coli*) following treatment of bladder urothelium cells with the three functional ingredients and the MIX at different concentrations;
- Figure 3 relates to expression histograms of IL-6 (A), IL-8 (B) and IL-1β (C) reported as fold change compared to the control (cells treated with medium conditioned only with the inflammatory stimulus, *E. coli*) following treatment of bladder urothelium cells with the three functional ingredients and the MIX at different concentrations;
- Figure 4 is a diagram illustrating the effect of the functional ingredients and the MIX on IL-6 levels in the different conditions tested (as reported in Table 9);
- Figure 5 is a diagram illustrating the effect of the functional ingredients and the MIX on the levels of IL-1β in the different conditions tested (as reported in Table 9); and
- Figure 6 is a diagram illustrating the effect of the functional ingredients and the MIX on IL-8 levels in the different conditions tested (as reported in Table 9).

### Detailed description of the invention

The invention relates to a composition comprising or consisting of palmitoylethanolamide (PEA), hemp seed oil and dry extract of maritime pine or proanthocyanidins, preferably procyanidins, for use in the treatment of pelvic pain.

The term "comprises" or "comprising" is used herein in the conventional meaning which does not exclude the presence of other ingredients in the composition, be they active ingredients, for example with anti-inflammatory or antioxidant activity or inert vehicles.

However, in the preferred embodiment, the composition consists of the aforementioned three ingredients, in its active part, thus excluding the presence of other active ingredients and preferably also the presence of inert vehicles or excipients.

The PEA used within the scope of the invention is preferably micronized PEA, commercially available, having a purity generally greater than 90% and preferably greater than or equal to 99% by weight.

Hemp seed oil, obtained from the seeds of *Cannabis sativa L*, used according to the invention and in the composition examples or dosage forms, mentioned below, is substantially free from phytocannabinoids such as cannabidiol (CBD), cannabigerol (CBG) and delta-9-tetrahedrocannabinol (THC).

The term "substantially free" is to be understood as meaning that the phytocannabinoid content is less than 2 mg/kg, in compliance with the legal requirements for products for food and nutraceutical use.

Furthermore, the composition according to the invention is substantially free from terpenes such as beta-caryophyllene (BCP).

Preferably, the invention uses a refined hemp seed oil for food use, which contains a mixture of fatty acids, such as:
- C16:0 palmitic acid for example from 4 to 9% by weight and/or
- C18:0 stearic acid for example 1-4% and/or
- C18:1 oleic acid for example 6-18% by weight and/or
- C18:2 linoleic acid for example 46-65% by weight and/or
- C18:3 alpha-linolenic acid for example 14-28% by weight and/or
- C18:3 gamma-linolenic acid for example ≤ 4% by weight and/or
- C18:4 stearidonic acid for example ≤ 2% by weight and/or
- C20:0 arachidic acid for example ≤ 1.5% by weight.

Preferably, hemp seed oil contains relevant quantities
- of total Ω-3 fatty acids (C18:3+C18:4) for example from 14 to 30% by weight and
- of S2-6 fatty acids (C18:2+C18:3) for example from 46 to 69% by weight.

Maritime pine extract is preferably a dry, powdered extract, preferably obtained from the bark of maritime pine (*Pinus pinaster Aiton*), preferably having a procyanidin content greater than 65% by weight and generally between 65 and 75% by weight.

Particularly preferred is the use of the dry extract of maritime pine called Pycnogenol^{®}.

The use of proanthocyanidins and in particular procyanidins, in place of the extract containing them, falls within the scope of the invention, the quantity of which by weight in the composition may be determined on the basis of the content of dry extract of maritime pine, taking into account a procyanidin content in the latter in accordance with the preferred value of 65 to 75% by weight.

In a preferred embodiment, the composition for use according to the invention comprises or consists of:
- from 100 to 1200 mg of PEA;
- from 2.5 to 30 mg of maritime pine dry extract or a corresponding amount of procyanidins, calculated on the basis of a procyanidin content in the aforementioned maritime pine dry extract of 65 to 75% by weight, and
- from 12.5 to 150 mg of hemp seed oil substantially free from phytocannabinoids.

In a preferred embodiment, the composition for use according to the invention comprises or consists of:
- from 200 to 800 mg of PEA;
- from 2.5 to 30 mg of maritime pine extract (or a corresponding amount of procyanidins, as indicated above) and
- from 12.5 to 125 mg of the aforementioned hemp seed oil.

According to a preferred embodiment, in the composition according to the invention, the weight ratio of PEA and hemp seed oil is from 8:1 to 100:1, preferably from 8:1 to 32:1. Preferably, the weight ratio of PEA to maritime pine dry extract is from 80:1 to 1:1, preferably from 80: 1 to 40:1.

The scope of the invention includes pharmaceutical or nutraceutical compositions in the form of dosage units, containing the quantities of active agents indicated above or multiples and submultiples thereof, preferably in the form of capsules, for example of soft gel.

The activity of the composition and dosage forms according to the invention was determined through the following experimental tests using an *in vitro* model which mimics the *in vivo* situation and presents the following advantages:
(i) use of human cell lines;
(ii) control of experimental conditions;
(iii) simplification of the model; and
(iv) compliance with the 3R principle.

In the following experimental tests, the composition according to the invention tested from time to time is identified with the term MIX.

### MATERIALS AND METHODS

### Description of the samples

The evaluation of the anti-inflammatory activity was carried out on the formulation (MIX) and its three components. The details are shown in **Table 1**.

**Table 1. Description of the analyzed samples.**

| **Sample** | **Format** |
|---|---|
| PLV PEA MICRONIZED (EZ00049678) | Powder |
| D.E. MARITIME PINE (EZ00050328) | Powder |
| REFINED EDIBLE HEMP OIL (EZ00049037) | Liquid |
| MIX OF PEA - HEMP OIL - PINE (VC139) | Powder |

### Determination of the titer in the three raw materials

The PEA content in the MICRONIZED PLV PEA raw material was determined by HPLC-DAD analysis, while the procyanidin content in the DRY MARITIME PINE EXTRACT (*Pinus pinaster* Aiton) was evaluated by the spectrophotometric method. The dosage of total lipids contained in HEMP OIL (*Cannabis sativa* L.) was measured by analysis with Gas-chromatography-FID.

### Bacterial strain

An aliquot of *Escherichia coli* (*E. coli*, ATCC^{®} 8739TM) was plated onto agar plates with Tryptic Soy Agar (TSA) medium and allowed to grow for 24 h. A single colony was picked with a sterile inoculating loop and inoculated into liquid Tryptic Soy Broth (TSB). After 24 h at 37 °C, the concentration of bacteria (colony forming units - CFU/mL) present in the inoculum was determined by densitometry and colony counting after plating serial dilutions on TSA agar plates. The growth of *E. coli* in liquid (broth) and solid (agar) medium was evaluated under aerobic conditions at 37 °C. A fresh inoculum was prepared before each experiment to ensure repeatability of the experimental conditions.

### In vitro innate immune system model

Human THP-1 monocytes (ATCC^{®} TIB-202TM) were maintained in THP-1 complete medium (THP-1 CCM) and cultured in a controlled atmosphere at 37 °C. Differentiation into macrophages was induced by incubation with phorbol myristate acetate (PMA) for 24 h. The culture medium was then replaced and the cells left with fresh medium for another 24 h before being exposed to the three functional ingredients, their MIX and the inflammatory stimulus, *E. coli.*

### Determination of the cytotoxicity of the three functional ingredients and their MIX

The cytotoxicity of the three functional ingredients and their MIX was evaluated on a THP-1 immune system model differentiated into macrophages. Based on the method of intake of the formulation, a digestion volume of 2 liters was considered in order to mimic as much as possible the physiological condition of the average volume of liquid contained in the stomach during the digestive process. Cytotoxicity was carried out by constructing a dose-response curve at increasing concentrations of the individual functional ingredients and the MIX in order to identify the maximum concentration usable for subsequent tests. The following concentrations were therefore tested on THP-1: from 50 µg/mL to 600 µg/mL for PEA, from 1.25 µg/mL to 15 µg/mL for DRY MARITIME PINE EXTRACT, from 6.25 µg/mL to 75 µg/mL for HEMP OIL and from 57.5 µg/mL to 690 µg/mL for the MIX. Based on the results of the dose-response curves, cell viability analysis was not performed at higher concentrations due to mortality caused by the mixture at the highest concentration tested. The concentrations used for subsequent experiments were selected in this concentration range. The three functional ingredients and the MIX were dissolved in THP-1 CCM in the absence of antibiotic and in the presence of 1.18 mg/mL of bile as surfactant. The suspensions were incubated under stirring overnight to increase the passage of the functional ingredients into solution and, the following day, the THP-1 cells were washed with Hank's Balanced Salt Solution (HBSS) and pre-treated for 2 h with the different concentrations of the individual functional ingredients and the MIX. At the end of this incubation, the pro-inflammatory stimulus *E. coli* (1 × 10⁶ CFU/mL) was added to the treated cells for a further 6 h. Cells treated only with *E. coli* were used as a negative control, while cells treated with 10 µM staurosporine were used as a positive cell death control. At the end of the 8 h of treatment, cell viability was assessed by ATP assay (PerkinElmer) to avoid any possible interference from the bacteria present that may occur using other tests such as MTS.

### Preparation of THP-1 conditioned medium for treatment of in vitro bladder urothelium model

Based on the results of the cytotoxicity experiments, the medium conditioned on THP-1 to be used to study the possible anti-inflammatory activity on the bladder urothelium was obtained by exposing for 2 h the differentiated THP-1 cells to the concentrations of the three functional ingredients and the MIX corresponding to 600, 400, 200, 100, 50 and 25 µg/mL for the MICRONIZED PEA, 15, 10, 5, 2.5, 1.25, 0.625 µg/mL for the DRY MARITIME PINE EXTRACT, 75, 50, 25, 12.5, 6.25, 3.125 µg/mL for HEMP OIL and 690, 460, 230, 115, 57.5 and 28.75 µg/mL for the MIX, respectively. At the end of this incubation, the cells were treated for a further 6 h with the inflammatory stimulus *E. coli* in the presence of the different treatments. Cells exposed only with the inflammatory stimulus in the absence of pre-treatment were used as a positive control of inflammation.

### Evaluation of the anti-inflammatory activity on an in vitro bladder urothelium model of the three functional ingredients and their MIX

The specific anti-inflammatory activity on the bladder urothelium of the three functional ingredients and the MIX was evaluated on an *in vitro* bladder urothelium epithelium composed of the T24 cell line (ATCC HTB-4TM). T24 cells were exposed for 4 h to conditioned media obtained by treating THP-1 cells differentiated into macrophages with different concentrations of the functional ingredients and MIX, and in the presence of *E. coli.* At the end of treatment, T24 cells were detached with trypsin, centrifuged, and lysed by sonication in lysis buffer. The supernatant obtained following centrifugation at 10,000 g for 10 min at 4 °C was stored at -80 °C. An extended analysis of the impact of functional ingredients and MIX on inflammation was performed using an assay for the determination of the transcription factor Nf-kB (Abcam) and the evaluation of the expression of pro-inflammatory cytokines with an array of 23 human cytokines (Raybiotech), following the manufacturer's instructions. The cytokines interleukin 1β (IL-1β), interleukin 6 (IL-6), and interleukin 8 (IL-8) were quantified by ELISA assay.

The results are reported expressed as fold change, calculated as the ratio between the values obtained from the respective treatments on the control of inflammation. As such, the fold change is adimensional.

### Determination of the titer in the three raw materials

The content of PEA in the MICRONIZED PEA powder, procyanidins in the DRY MARITIME PINE EXTRACT and total lipids in the HEMP OIL was determined respectively by HPLC-DAD, spectrophotometric analysis and Gas-chromatography-FID and compared with the titer declared in the technical data sheets of the three raw materials. As reported in **Table 2**, the measured content is comparable with that declared for all three raw materials.

**Table 2. Comparison between the measured titer and the declared titer of PEA, procyanidins and total lipids in the three raw materials.**

| **Functional ingredient** | **Content measured (%)** | **Content declared (%)** |
|---|---|---|
| MICRONIZED PEA (PEA) | 91.1 ± 9.8 | ≥ 99 |
| D.E. MARITIME PINE (Procyanidins) | 73.6 ± 0.8 | 65< x <75 |
| HEMP OIL (total lipids) | 99.9 | 100 |

### Determination of the impact of the three functional ingredients and their MIX on the viability of the in vitro immune system model

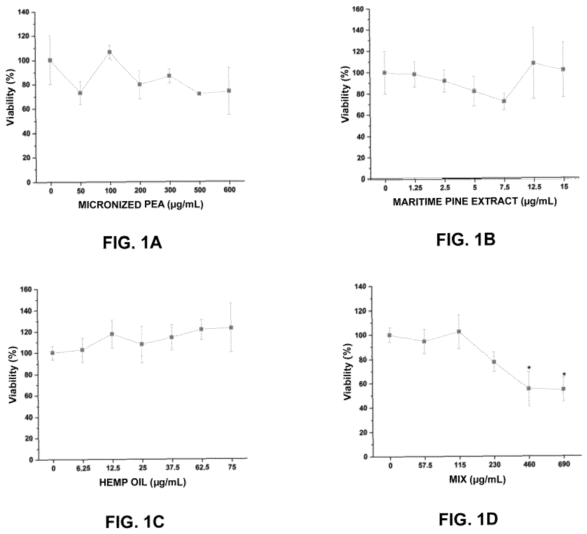

The cytotoxic activity of the three functional ingredients and the MIX in the presence of the inflammatory stimulus *E. coli* was evaluated on an *in vitro* innate immune system model, following the protocol described in the Materials and Methods paragraph. The three functional ingredients do not induce negative effects on the viability of macrophages, as shown in **Figure 1A****, B, C** and reported in **Table 3, 4, 5**. The MIX has a dose-dependent effect on the cell viability of differentiated THP-1, reaching a plateau with a viability value lower than 70% (54.5 ± 9.9%) compared to the negative control (cells treated with *E. coli)* at a concentration of 690 µg/mL (1380 mg) (**Figure 1D****, Table 6**). Based on the viability results, the maximum concentration of MIX used for the experiments was 690 µg/mL (1380 mg), corresponding to MICRONIZED PEA 600 µg/mL (1200 mg), MARITIME PINE EXTRACT 15 µg/mL (30 mg) and HEMP OIL 75 µg/mL (150 mg). Concentrations greater than the maximum concentration tested were not investigated due to the effect on viability induced by the MIX, while lower concentrations of MIX and functional ingredients were studied for their anti-inflammatory capacity.

**Table 3. Cell viability of differentiated THP-1 following treatment with MICRONIZED PEA.**

| M**ICRONIZED PEA (mg)** | **MICRONIZED PEA (µg/ML)** | **Viability (%)** |
|---|---|---|
| 0 (Control) | 0 (Control) | 100 ± 20.2 |
| 100 | 50 | 73.2 ± 9.5 |
| 200 | 100 | 106.5 ± 5.6 |
| 400 | 200 | -79.6 ± 11.6 |
| 600 | 300 | 86.5 ± 5.9 |
| 1000 | 500 | 71.9 ± 1.3 |
| 1200 | 600 | 73.9 ± 19.5 |

**Table 4. Cell viability of differentiated THP-1 following treatment with MARITIME PINE EXTRACT.**

| **MARITIME EXTRACT (mg)** | **PINEMARITIME PINE EXTRACT (µg/mL)** | **Viabilty (%)** |
|---|---|---|
| 0 (Control) | 0 (Control) | 100 ± 20.2 |
| 2.5 | 1.25 | 98.3 ± 12.1 |
| 5 | 2.5 | 91.7 ± 10.6 |
| 10 | 5 | 82.2 ± 14.0 |
| 15 | 7.5 | 72.4 ± 7.9 |
| 25 | 12.5 | 108.2 ± 33.5 |
| 30 | 15 | 101.8 ± 25.7 |

**Table 5. Cell viability of differentiated THP-1 following treatment with HEMP OIL.**

| **HEMP OIL(mg)** | **HEMP OIL (µg/mL)** | **Viability (%)** |
|---|---|---|
| 0 (Control) | 0 (Control) | 100 ± 6.1 |
| 12.5 | 6.25 | 102.8 ± 11.1 |
| 25 | 12.5 | 117.7 ± 13.3 |
| 50 | 25 | 107.8 ± 17.1 |
| 75 | 37.5 | 114.1 ± 12.1 |
| 125 | 62.5 | 121.8 ± 9.4 |
| 150 | 75 | 122.9 ± 23.2 |

**Table 6. Cell viability of differentiated THP-1 following treatment with the MIX.**

| **MIX (mg)** | **MIX (µg/mL)** | **Viability (%)** |
|---|---|---|
| 0 (Control) | 0 (Control) | 100 ± 6.1 |
| 115 | 57.5 | 94.5 ± 10.0 |
| 230 | 115 | 102.5 ± 14.0 |
| 460 | 230 | 77.5 ± 7.9 |
| 920 | 460 | 55.2 ± 14.1* |
| 1380 | 690 | 54.5 ± 9.9* |

| | | |
|---|---|---|
| *cell viability < 70% | | |

### Impact of conditioned media on inflammation of the bladder urothelium in vitro

The specific anti-inflammatory activity on the bladder urothelium of the three functional ingredients and MIX was evaluated by exposing T24 cells to conditioned media for 4 h. The anti-inflammatory response was investigated by evaluating the effect of the treatments on the nuclear levels of the transcription factor Nf-kB, analysis of the release levels of 23 cytokines and ELISA assays for the cytokines IL-6, IL-8 and IL-1β, as more involved in inflammatory processes.

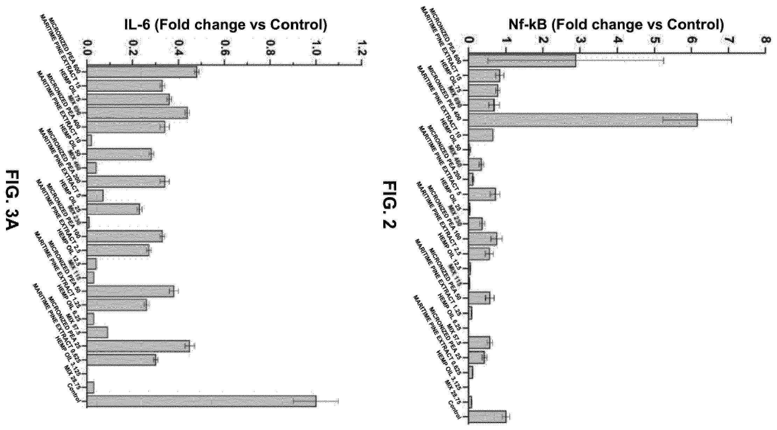

As shown in **Figure 2** and **Table 7**, Nf-kB levels are reduced following treatment with DRY MARITIME PINE EXTRACT, HEMP OIL and MIX, demonstrating their effectiveness in reducing the translocation of this pro-inflammatory factor within of the cell nucleus, an event that is observed following an inflammatory process. Treatment with MICRONIZED PEA reduces the nuclear translocation of the transcription factor NF-κB starting from 25 µg/mL (50 mg) up to 200 µg/mL (400 mg). The MIX proved to be more effective in reducing the nuclear concentration of Nf-kB compared to the single functional ingredients at the MIX concentration equal to 115 µg/mL equal to 230 mg and at the maximum concentration used (690 µg/mL equal to 1380 mg).

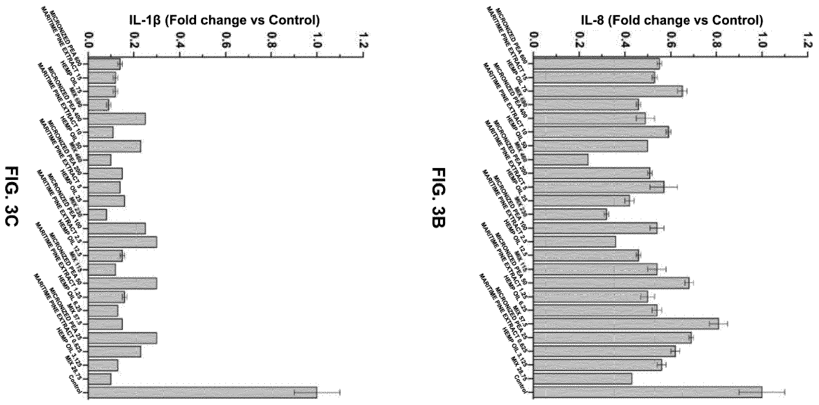

**Table 8** shows the expression levels of three cytokines that had a significant change in the treated compared to the control: GRO alpha, GRO a/b/g and GM-CSF. These cytokines are involved in the inflammatory response and in particular in the recruitment and activation of immune system cells, ensuring they reach the site of inflammation. From these data it may be seen that the combination of the three functional ingredients studied is effective in reducing the inflammation triggered by the presence of the pathogen *E. coli.* The synergistic action of MICRONIZED PEA, MARITIME PINE EXTRACT and HEMP OIL is particularly evident when the MIX is used at concentrations of 57.5 µg/mL (115 mg) (with a value of 0.96 ± 0.00 per GRO a/b/g), 115 µg/mL (230 mg) (with a value of 0.78 ± 0.01 for GRO alpha), 230 µg/mL (460 mg) (with a value of 0.65 ± 0.02, 0.51 ± 0.05, 0.57 ± 0.00 for GRO alpha, GRO a/b/g and GM-CSF respectively), 460 µg/mL (920 mg) (with a value of 0.58 ± 0.06, 0.58 ± 0.04, 0.58 ± 0.00 for GRO alpha, GRO a/b/g and GM-CSF respectively) and 690 µg/mL (1380 mg) (with a value of 0.99 ± 0.03 for GRO alpha). The individual functional ingredients used individually at the corresponding concentrations do not have the same ability to attenuate the inflammatory process. The cytokines IL-6, IL-8 and IL-1β, being more involved in inflammatory processes, were studied in more depth by ELISA assay and the results are reported below. As shown in **Figure 3** and **Table 9**, treatment with the individual functional ingredients and their MIX causes a reduction in the expression of all three cytokines studied. In particular, MIX determines the lowest expression levels of all three cytokines compared to the individual functional ingredients at a concentration of 230 µg/mL (460 mg) (with a value of 0.01 ± 0.00, 0.32 ± 0.01 and 0.08 ± 0.00 for IL-6, IL-8 and IL-1β respectively), suggesting a synergistic action of MICRONIZED PEA, MARITIME PINE EXTRACT and HEMP OIL. At 115 µg/mL (230 mg), MIX is most effective in reducing IL-6 (with a value of 0.03 ± 0.00) and IL-1β (with a value of 0.12 ± 0.00 and IL-1β) (**Figure 3** and **Table 9**). The synergistic action at the highest concentrations of MIX tested translates into the reduction of IL-8 expression with a value of 0.24 ± 0.00 at the concentration of 460 µg/mL (920 mg) and 0.46 ± 0.01 at the concentration of 690 µg/mL (1380 mg), and in the reduction of IL-1β expression with a value of 0.10 ± 0.00 at the concentration of 460 µg/mL (920 mg) and 0.09 ± 0.01 at the concentration of 690 µg/mL (1380 mg) (**Figure 3B**, **C** and **Table 9**).

**Table 7. Nuclear levels of the transcription factor Nf-kB expressed as fold change compared to the control (cells treated with medium conditioned only with the inflammatory stimulus, E. coli) following treatment of bladder urothelium cells with the three functional ingredients and the MIX at the different concentrations. *lower values than individual functional ingredients**

| | | | **Nf-kB** (**Fold change *vs* Control**) | |
|---|---|---|---|---|
| | **Treatment (mg)** | **Treatment (µg/mL)** | **Average** | **SD** |
| **Condition 1** | MICRONIZED PEA 1200 | MICRONIZED PEA 600 | 2.89 | 2.37 |
| | MARITIME PINE EXTRACT 30 | MARITIME PINE EXTRACT 15 | 0.83 | 0.11 |
| | HEMP OIL 150 | HEMP OIL 75 | 0.78 | 0.06 |
| | MIX 1380 | MIX 690 | 0.68* | 0.14 |
| | | | | |
| **Condition 2** | MICRONIZED PEA 800 | MICRONIZED PEA 400 | 6.16 | 0.92 |
| | MARITIME PINE EXTRACT 20 | MARITIME PINE EXTRACT 10 | 0.65 | 0.00 |
| | HEMP OIL 100 | HEMP OIL 50 | 0.02 | 0.04 |
| | MIX 920 | MIX 460 | 0.35 | 0.06 |
| | | | | |
| **Condition 3** | MICRONIZED PEA 400 | MICRONIZED PEA 200 | 0.12 | 0.02 |
| | MARITIME PINE EXTRACT 10 | MARITIME PINE EXTRACT 5 | 0.71 | 0.13 |
| | HEMP OIL 50 | HEMP OIL 25 | 0.04 | 0.01 |
| | MIX 460 | MIX 230 | 0.37 | 0.07 |
| | | | | |
| **Condition 4** | MICRONIZED PEA 200 | MICRONIZED PEA 100 | 0.75 | 0.15 |
| | MARITIME PINE EXTRACT 5 | MARITIME PINE EXTRACT 2.5 | 0.56 | 0.11 |
| | HEMP OIL 25 | HEMP OIL 12.5 | 0.05 | 0.01 |
| | MIX 230 | MIX 115 | 0.03* | 0.00 |
| | | | | |
| **Condition 5** | MICRONIZED PEA 100 | MICRONIZED PEA 50 | 0.57 | 0.11 |
| | MARITIME PINE EXTRACT 2.5 | MARITIME PINE EXTRACT 1.25 | 0.09 | 0.01 |
| | HEMP OIL 12.5 | HEMP OIL 6.25 | 0.00 | 0.00 |
| | MIX 115 | MIX 57.5 | 0.57 | 0.07 |
| | | | | |
| **Condition 6** | MICRONIZED PEA 50 | MICRONIZED PEA 25 | 0.43 | 0.06 |
| | MARITIME PINE EXTRACT 1.25 | MARITIME PINE EXTRACT 0.625 | 0.11 | 0.01 |
| | HEMP OIL 6.25 | HEMP OIL 3.125 | 0.01 | 0.00 |
| | MIX 57.5 | MIX 28.75 | 0.08 | 0.01 |

**Table 8. Expression of GRO alpha, GRO a/b/g and GM-CSF reported as fold change compared to the control (cells treated with medium conditioned only with the inflammatory stimulus, E. coli) following treatment of bladder urothelium cells with the three functional ingredients and the MIX at the different concentrations. *lower values than individual functional ingredients**

| | | | G**RO alpha (Fold change *vs* Control)** | | G**RO a/b/g (Fold change *vs* Control)** | | **GH-CSF (Fold change *vs* Control)** | |
|---|---|---|---|---|---|---|---|---|
| | **Treatment (mg)** | **Treatment** | **Average** | **SD** | **Average** | **SD** | **Average** | **SD** |
| **Condition 1** | MICRONIZED PEA | MICRONIZED PEA | 1.49 | 0.06 | 1.14 | 0.04 | 1.01 | 0.08 |
| | 1200 | 600 | | | | | | |
| | MARITIME PINE EXTRACT | MARITIME PINE EXTRACT 15 | 1.79 | 0.03 | 1.91 | 0.02 | 1.39 | 0.02 |
| | HEMP OIL 150 | HEMP OIL 75 | 1.43 | 0.03 | 1.56 | 0.06 | 1.21 | 0.02 |
| | MIX 1380 | MIX 690 | 0.99* | 0.03 | 1.19 | 0.06 | 1.10 | 0.21 |
| | | | | | | | | |
| **Condition 2** | MICRONIZED PEA | MICRONIZED PEA | 0.79 | 0.04 | 0.92 | 0.08 | 1.1 | 0.11 |
| | 800 | 400 | | | | | | |
| | MARITIME PINE EXTRACT | MARITIME PINE EXTRACT 10 | 0.71 | 0.16 | 0.97 | 0.13 | 0.81 | 0.14 |
| | HEMP OIL 100 | HEMP OIL 50 | 1.04 | 0.03 | 0.98 | 0.05 | 1.56 | 0.09 |
| | MIX 920 | MIX 460 | 0.58* | 0.06 | 0.58* | 0.04 | 0.58* | 0.00 |
| | | | | | | | | |
| **Condition 3** | MICRONIZED PEA | MICRONIZED PEA | 1.28 | 0.05 | 0.81 | 0.23 | 0.9 | 0.26 |
| | 400 | 200 | | | | | | |
| | MARITIME PINE | MARITIME PINE EXTRACT | 1 | 0.09 | 1.22 | 0.07 | 1.19 | 0.04 |
| | HEMP OIL 50 | HEMP OIL 25 | 0.9 | 0.13 | 0.88 | 0.04 | 1.36 | 0.01 |
| | MIX 460 | MIX 230 | 0.65* | 0.02 | 0.51* | 0.05 | 0.57* | 0.00 |
| | | | | | | | | |
| **Condition 4** | MICRONIZED PEA | MICRONIZED PEA | 1.23 | 0.13 | 0.58 | 0.37 | 0.21 | 0.26 |
| | 200 | 100 | | | | | | |
| | MARITIME PINE EXTRACT | MARITIME PINE EXTRACT 2.5 | 1.69 | 0.28 | 1.71 | 0.27 | 1.82 | 0.16 |
| | HEMP OIL 25 | HEMP OIL 12.5 | 1.17 | 0.09 | 1.17 | 0.08 | 1.71 | 0.02 |
| | MIX 230 | MIX 115 | 0.78* | 0.01 | 0.72 | 0.02 | 0.79 | 0.01 |
| | | | | | | | | |
| **Condition 5** | MICRONIZED PEA | MICRONIZED PEA | 0.91 | 0.03 | 1 | 0.08 | 1.15 | 0.11 |
| | 100 | 50 | | | | | | |
| | MARITIME PINE EXTRACT | MARITIME PINE EXTRACT 1.25 | 1.75 | 0.16 | 1.44 | 0.22 | 1.29 | 0.14 |
| | HEMP OIL 12.5 | HEMP OIL 6.25 | 1.23 | 0.03 | 1.16 | 0.07 | 1.47 | 0.05 |
| | MIX 115 | MIX 57.5 | 0.97 | 0.09 | 0.96* | 0.00 | 1.31 | 0.05 |
| | | | | | | | | |
| **Condition 6** | MICRONIZED PEA | MICRONIZED PEA | 1.17 | 0.01 | 1.13 | 0.00 | 1.19 | 0.3 |
| | MARITIME PINE EXTRACT 1.25 | MARITIME PINE EXTRACT 0.625 | 0.66 | 0.03 | 0.77 | 0.07 | 1.55 | 0.07 |
| | HEMP OIL 6.25 | HEMP OIL 3.125 | 1.19 | 0.04 | 1.2 | 0.07 | 1.52 | 0.05 |
| | MIX 57.5 | MIX 28.75 | 1.33 | 0.15 | 1.35 | 0.1 | 1.39 | 0.1 |

**Table 9. Expression of IL-6, IL-8 and IL-1β reported as fold change compared to the control (cells treated with medium conditioned only with the inflammatory stimulus, E. coli) following treatment of bladder urothelium cells with the three functional ingredients and the MIX at the different concentrations. *lower values than individual functional ingredients**

| | | | **IL-6 (Fold change *vs* Control)** | | **IL-8 (Fold change *vs* Control)** | | **IL-1β (Fold change *vs* Control)** | |
|---|---|---|---|---|---|---|---|---|
| | **Treatment (mg)** | **Treatment** | **Average** | **SD** | **Average** | **SD** | **Average** | **SD** |
| **Condition 1** | MICRONIZED PEA | MICRONIZED PEA | 0.48 | 0.01 | 0.55 | 0.01 | 0.14 | 0.01 |
| | 1200 | 600 | | | | | | |
| | MARITIME PINE EXTRACT | MARITIME PINE EXTRACT 15 | 0.33 | 0.01 | 0.53 | 0.01 | 0.12 | 0.01 |
| | HEMP OIL 150 | HEMP OIL 75 | 0.36 | 0.01 | 0.65 | 0.02 | 0.12 | 0.01 |
| | MIX 1380 | MIX 690 | 0.44 | 0.01 | 0.46* | 0.01 | 0.09* | 0.01 |
| | | | | | | | | |
| **Condition 2** | MICRONIZED PEA | MICRONIZED PEA | 0.34 | 0.02 | 0.49 | 0.04 | 0.25 | 0.00 |
| | 800 | 400 | | | | | | |
| | MARITIME PINE EXTRACT | MARITIME PINE EXTRACT 10 | 0.02 | 0.00 | 0.59 | 0.01 | 0.11 | 0.00 |
| | HEMP OIL 100 | HEMP OIL 50 | 0.28 | 0.01 | 0.50 | 0.00 | 0.23 | 0.00 |
| | MIX 920 | MIX 460 | 0.04 | 0.00 | 0.24* | 0.00 | 0.10* | 0.00 |
| | | | | | | | | |
| **Condition 3** | MICRONIZED PEA | MICRONIZED PEA | 0.34 | 0.02 | 0.51 | 0.01 | 0.15 | 0.00 |
| | 400 | 200 | | | | | | |
| | MARITIME PINE EXTRACT | MARITIME PINE EXTRACT 5 | 0.07 | 0.00 | 0.57 | 0.06 | 0.14 | 0.00 |
| | HEMP OIL 50 | HEMP OIL | 0.23 | 0.01 | 0.42 | 0.02 | 0.16 | 0.00 |
| | | 25 | | | | | | |
| | MIX 460 | MIX 230 | 0.01* | 0.00 | 0.32* | 0.01 | 0.08* | 0.00 |
| | | | | | | | | |
| **Condition 4** | MICRONIZED PEA | MICRONIZED PEA | 0.33 | 0.01 | 0.54 | 0.03 | 0.25 | 0.00 |
| | 200 | 100 | | | | | | |
| | MARITIME PINE EXTRACT | MARITIME PINE EXTRACT 2.5 | 0.27 | 0.01 | 0.36 | 0.00 | 0.30 | 0.00 |
| | HEMP OIL 25 | HEMP OIL | 0.04 | 0.00 | 0.46 | 0.01 | 0.15 | 0.01 |
| | MIX 230 | MIX 115 | 0.03* | 0.00 | 0.54 | 0.04 | 0.12* | 0.00 |
| | | | | | | | | |
| **Condition 5** | MICRONIZED PEA | MICRONIZED PEA | 0.38 | 0.02 | 0.68 | 0.02 | 0.30 | 0.00 |
| | 100 | 50 | | | | | | |
| | MARITIME PINE EXTRACT | MARITIME PINE EXTRACT 1.25 | 0.26 | 0.01 | 0.50 | 0.03 | 0.16 | 0.01 |
| | HEMP OIL 12.5 | HEMP OIL 6.25 | 0.03 | 0.00 | 0.54 | 0.02 | 0.13 | 0.00 |
| | MIX 115 | MIX 57.5 | 0.09 | 0.00 | 0.81 | 0.04 | 0.15 | 0.00 |
| | | | | | | | | |
| **Condition 6** | MICRONIZED PEA | MICRONIZED PEA | 0.45 | 0.02 | 0.69 | 0.01 | 0.30 | 0.00 |
| | 50 | 25 | | | | | | |
| | MARITIME PINE EXTRACT | MARITIME PINE EXTRACT | 0.30 | 0.01 | 0.62 | 0.02 | 0.23 | 0.00 |
| | HEMP OIL 6.25 | HEMP OIL | 0.00 | 0.00 | 0.56 | 0.02 | 0.13 | 0.00 |
| | | 3.125 | | | | | | |
| | MIX 57.5 | MIX 28.75 | 0.03 | 0.00 | 0.43 | 0.00 | 0.10 | 0.00 |

### DISCUSSION and CONCLUSIONS

At the level of the bladder, the response to inflammation caused by pathogenic microorganisms (e.g. *Escherichia coli*) is mainly mediated by resident macrophages, present beneath the urothelium. The cytokine environment or milieu created by the resident macrophages is in turn responsible for the inflammation of the urothelial cells. The *in vitro* model used is based on two human cell lines representing the innate immune system (THP-1) and the urothelium (T24). The monocytic/macrophage cell line THP-1 represents one of the most used *in vitro* models for studying the anti-inflammatory activity of formulations. Literature studies have shown that THP-1s respond to stimuli in a similar way to their *in vivo* counterparts. T24 represent the cell line most used for studying the efficacy and safety of formulations and drugs at the urothelial level. In *in vitro* models it is also possible to control the experimental conditions, reducing intra- and inter-experimental variability.

The results obtained demonstrated that the mixture composed of MICRONIZED PEA, MARITIME PINE EXTRACT and HEMP OIL, at different concentrations, exerts a synergistic anti-inflammatory action which translates into greater effectiveness compared to that exerted by its individual components. The composition according to the invention reduces:
- the nuclear content of the transcription factor Nf-kB at concentrations of 115 µg/mL (230 mg) and 690 µg/mL (1380 mg);
- the expression of the pro-inflammatory cytokine GRO-alpha in the range between the concentrations 115 µg/mL (230 mg) and 690 µg/mL (1380 mg);
- the expression of the pro-inflammatory cytokine GRO a/b/g at concentrations of 57.5 µg/mL (115 mg), 230 µg/mL (460 mg) and 460 µg/mL (920 mg);
- the expression level of the cytokine GM-CSF at concentrations of 230 µg/mL (460 mg) and 460 µg/mL (920 mg);
- the expression of IL-1β in the range between the concentrations of 115 µg/mL (230 mg) and 690 µg/mL (1380 mg);
- the expression of IL-6 in the range between the concentrations of 115 µg/mL (230 mg) and 230 µg/mL (460 mg);
- the expression of IL-8 in the range between the concentrations 230 µg/mL (460 mg) and 690 µg/mL (1380 mg).

The anti-inflammatory efficacy of the formulation is particularly evident for the cytokines IL-8 and II-1β. Cells of the bladder urothelium express but do not normally secrete IL-8. The release of interleukin 8 occurs in response to inflammatory events affecting the urinary tract caused for example by pathogenic microorganisms, as in the case of *E. coli.* The greater reduction in the levels of this cytokine, following treatment with the mixture studied compared to the individual functional ingredients, supports the hypothesis of the presence of a synergistic action of the formulation at the level of the bladder urothelium.

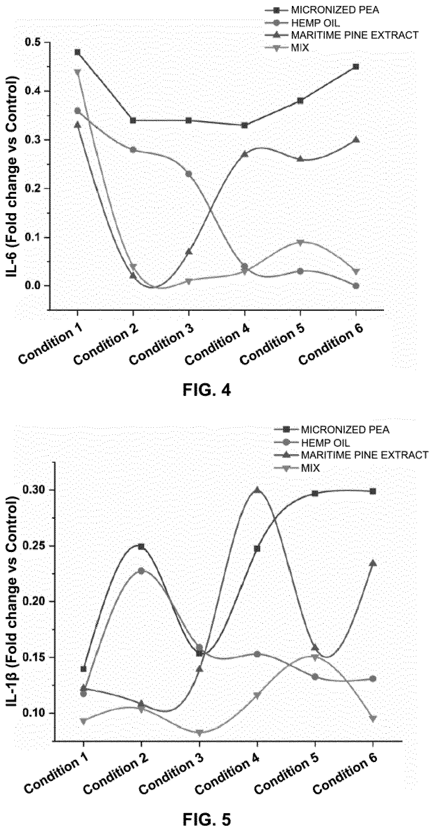

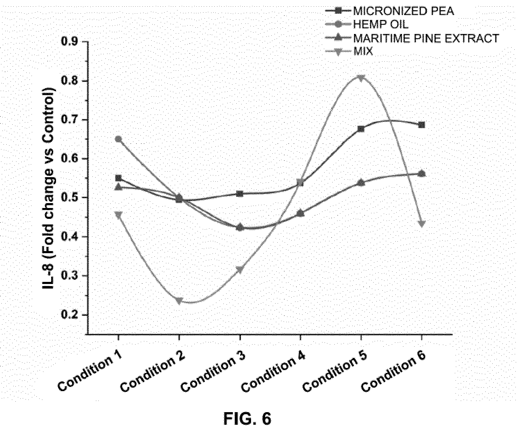

From the data obtained it may be seen that MICRONIZED PEA has a dose-independent anti-inflammatory effect on the expression of IL-6, while MARITIME PINE EXTRACT and HEMP OIL have a dose-dependent but opposite effect. The first contributes to the anti-inflammatory action at the highest concentrations tested (condition 2 and 3 which correspond to concentrations of 10 and 5 µg/mL - 20 and 10 mg), the second instead acts more when used at the lowest concentrations (condition 4, 5 and 6 which correspond to 12.5, 6.25 and 3.125 µg/mL - 25, 12.5 and 6.25 mg) (**Fig. 4**). The synergistic action of MARITIME PINE EXTRACT and HEMP OIL within the MIX in the reduction of IL-6 is greater in condition 3 (fold change 0.01), which corresponds to a concentration of the MIX equal to 230 µg /mL (460 mg). Similar contributions were also observed for IL-1β (**Fig. 5**). The three active ingredients taken individually reduce IL-8 in a dose-independent manner, while combined in a mixture they create a synergistic effect corresponding to an increased anti-inflammatory activity for IL-8 under conditions 1, 2 and 3 which correspond to a MIX concentration of 690, 460 and 230 µg/mL (1380, 920 and 460 mg) (**Fig. 6**). In light of the data obtained, the composition of the invention is characterized by an anti-inflammatory activity at the level of the urothelium which derives from the synergy of the functional ingredients contained therein. Considering that the composition of the invention will be administered orally, it will undergo exposure to the digestive process and colonic fermentation by the microbial community or intestinal microflora. Among the different tasks carried out by this diverse community of microorganisms is the fermentation of compounds not processed during digestion, such as fibers. Furthermore, the literature reports the role of fermentation in the inactivation or activation of numerous active ingredients including procyanidins which, post-fermentation, appear to increase in the blood circulation with a consequent possible increase in their anti-inflammatory action. Therefore, the action of MARITIME PINE EXTRACT and its contribution to the composition may be amplified by the microbiota during the digestive process.

Based on the results obtained, the present invention provides a composition containing active and natural agents, usable in the food and nutraceutical field for use in the treatment of acute, cyclic and chronic pelvic pain, which has a broad spectrum of action on cytokines inflammatory processes involved in the generation of pain and which presents a substantially improved and synergistic effect compared to the action of the individual active agents used.

Also within the scope of the invention is the composition for the above-mentioned use as an adjuvant in the treatment of inflammatory and neuroinflammatory pelvic pathologies in women and men, such as Cystitis (acute, recurrent, chronic; bacterial or abacterial), Interstitial cystitis, Calculosis, Ureteritis, Urethritis, Urethral syndrome, Nephritis (pyelonephritis, glomerulonephritis, tubulointerstitial nephritis), Bladder pain syndrome (BPS), Chronic pelvic pain syndrome (CPPS), Neurological bladder, Bladder neck sclerosis, Prostatitis (acute, recurrent, chronic; bacterial or abacterial), Prostadymia, Primary dysmenorrhea, Endometriosis, Adenomyosis, Pelvic inflammatory disease, Uterine fibromatosis, Functional somatic syndromes (FSS's): vulvar vestibulitis and vulvodynia, Dyspareunia, Cervicitis, Endometritis, Pelvic neoplasms.

The invention is implemented by dosage forms for oral administration such as capsules in particular. Preferred dosage forms contain the quantities of MIX described in Table 9 and corresponding to conditions 1 to 5. Particularly preferred is the creation of a dosage form corresponding to condition 3 and containing:

| | |
|---|---|
| - Micronized PEA | 400 mg |
| - maritime pine extract | 10 mg |
| - hemp oil | 50 mg, |

it being understood that the preferred features previously stated apply to the above-mentioned ingredients. In this dosage form, oral administration of 1 to 3 capsules/day may be envisaged.

## Claims

1. Pharmaceutical or nutraceutical composition comprising palmitoylethanolamide (PEA), dry extract of maritime pine and hemp seed oil substantially free from phytocannabinoids, for use in the treatment of pelvic pain.

2. Composition for use according to claim 1, comprising:
- from 100 to 1200 mg of PEA,
- from 2.5 to 30 mg of dry extract of maritime pine in quantities from 1.6 to 4.2 mg, and
- from 12.5 to 150 mg of hemp seed oil substantially free from phytocannabinoids.

3. Composition for use according to claim 1 or 2, **characterized in that** it comprises or consists of:
- from 200 to 800 mg of PEA,
- from 2.5 to 30 mg of dry extract of maritime pine, and
- from 12.5 to 125 mg of said hemp oil.

4. Composition for use according to any one of claims 1 to 3, **characterized in that** said hemp oil comprises a total amount of S2-3 fatty acids from 14 to 30% by weight and a total amount of Ω-6 fatty acids from 46 to 69% by weight.

5. Composition for use according to any one of claims 1 to 4, **characterized in that** said dry extract of maritime pine has a quantity of proanthocyanidins, preferably procyanidins, not less than 65% by weight.

6. Composition for use according to any one of the preceding claims, wherein the weight ratio of PEA and hemp oil is from 8:1 to 100:1, preferably from 8:1 to 32:1.

7. Composition for use according to any one of claims 1 to 6, wherein the weight ratio of PEA and dry extract of maritime pine is from 80:1 to 1:1, preferably from 40:1 to 1:1.

8. Composition for use according to any one of claims 1 to 7 in the form of dosage units for oral administration.

9. Composition for use according to any one of the preceding claims, wherein the pelvic pain is caused by an inflammatory and neuro-inflammatory pelvic pathology selected from the group consisting of Cystitis (acute, recurrent, chronic; bacterial or abacterial), Interstitial cystitis, Calculosis, Ureteritis, Urethritis, Urethral syndrome, Nephritis (pyelonephritis, glomerulonephritis, tubulointerstitial nephritis), Bladder pain syndrome (BPS), Chronic pelvic pain syndrome (CPPS), Neurological bladder, Bladder neck sclerosis, Prostatitis (acute, recurrent, chronic; bacterial or abacterial), Prostadymia, Primary dysmenorrhea, Endometriosis, Adenomyosis, Pelvic inflammatory disease, Uterine fibromatosis, Functional somatic syndromes (FSS's): vulvar vestibulitis and vulvodynia, Dyspareunia, Cervicitis, Endometritis, Pelvic neoplasms.
